# EUROPEAN PATENT APPLICATION

(11) **EP 1 428 550 A1**
(43) Date of publication of application: **16.06.2004**
(21) Application number: 02425768.5
(22) Date of filing: 13.12.2002
(51) Int. Cl.: A61N 1/05

(54) **Single pacemaker catheter electrode for the biventricular cardiac electrostimulation**

(71) Applicant: P.A. & M. S.p.A., 00198 Roma (IT)
(72) Inventor: De Bellis, Ferruccio, 00198 Roma (IT)
(74) Representative: Mariani, Giulio

(57) **Abstract**

A single pacemaker catheter electrode for the biventricular cardiac electrostimulation by an assisted cardiac stimulation device by the insertion of its stimulation tip into the right ventricle of the heart without the need of using a second catheter electrode directed to stimulate the left ventricle, including a stimulation screw having a penetration tip brought to the distal end of the catheter electrode which is such as to allow the electrostimulation screw to be driven from the outside in order to deeply penetrate into the ventricular septum separating the two ventricles.

## Description

The present invention relates to a single pacemaker catheter electrode for the biventricular cardiac electrostimulation.

In particular cases of the cardiac electrostimulation, as known, it is necessary to carry out a biventricular electrostimulation, i.e. a direct stimulation of both ventricles. This is the case when it is essential a perfect synchronism between the contractions of both right and left ventricles.

The biventricular stimulation requires currently, in addition to the atrial catheter electrode, the positioning of two ventricular catheter electrodes, one in the right ventricle and the other, for the left ventricle, in a superficial branch of the great cardiac vein which is reached by the catheter electrode through the coronary sinus. However, the positioning of the second electrode for the stimulation of the left ventricle during a biventricular stimulation is a very complex operation. In fact, as already mentioned, as the second electrode is put into a superficial branch of the great cardiac vein, it has to be positioned across the coronary sinus. Such positioning requires the use of special insertion instruments as well as a contrast medium for the radiological control of the operation.

Following the second ventricular electrode insertion, serious postoperative complications due to micro- and macro-displacements of the electrode tip and great increase of the cardiac threshold current which is often non-compatible with the characteristics of the implanted pacemaker are not infrequent.

To sum up, performing an implantation for the biventricular electrostimulation is a complex operation and not free from considerable troubles because of the presence of an atrial electrode and two ventricular catheter electrodes as well as the complex technique of implanting the second of the latter.

The present invention seeks to solve the problem mentioned above by providing a catheter electrode conceived and constructed so that its insertion into the right ventricle permits the biventricular electrostimulation without the need of using a second catheter electrode directed to stimulate the left ventricle. In other words the catheter electrode according to the invention is such that its insertion alone into the right ventricle permits the biventricular electrostimulation which was so far possible only by the use of two catheter electrodes associated as usual to the atrial electrode.

The electrode according to the invention is a pull-out screw catheter electrode in which the distal screw end can axially come out by about 8-10 mm instead of 1,5 mm in the axial direction as the currently used electrodes. For the reasons discussed thereafter the feed of the distal end should be graded. The distal end of the electrode according to the invention includes an inside threaded pipe within which a worm screw with an outside thread conjugated to the inside thread of such pipe is moved. Secured to the worm screw is a special screw for the stimulation which has a length equal to 8-10 mm in the axial direction and enters the interventricular septum, as better discussed thereafter. The worm screw can be rotated inside the pipe by the rotation of the so-called spindle which is driven by the operator from the outside. As the implantation is ready, the stimulating end is positioned in contact with the wall of the interventricular septum inside the right ventricle in a high "subtricuspid" position, i.e. under the tricuspid valve, then the spindle of the catheter electrode is driven so as to cause the worm screw integral therewith to rotate. The rotation of the worm screw inside the threaded pipe will cause it to move forward inside the latter, thus causing the stimulation tip to come out and simultaneously to rotate about its own axis. The thread pitch is preferably selected so that the feed of the helical stimulating tip by one millimetre measured axially corresponds to a whole number of revolutions of the spindle. The stimulating tip is then fed by at least four millimetres inside the muscle of the interventricular septum, performing from time to time suitable measurements from the outside to control the position of the tip of the catheter electrode and the electrical value of the stimulation threshold. Once the correct position of the stimulating tip in the interventricular septum is ascertained, the stimulating tip is caused to further feed into the septum by rotating the spindle from the outside.

Such feed will be so graded as to be carried out by one millimetre at a time, performing every millimetre feed electrical stimulation tests by discrete pulses until the monitor of the electrocardiograph displays a biventricular electrostimulation, i.e. a stimulation which is obtained by the same pulse for both ventricles.

The catheter electrode for biventricular electrostimulation by only one catheter according to the invention will now be described in detail with reference to the accompanying drawings in which:
Fig. 1 is a schematic view of a pacemaker implantation using the catheter electrode according to the invention;
Fig. 2 is a schematic view only of the catheter electrode and the way it is put into the heart;
Fig. 3 is a detailed view, in enlarged scale, of the position of the end portion of the catheter electrode of Fig. 2 in the heart;
Fig. 4 is a section of the catheter electrode according to the invention provided with a control knob at its outer end, and with the inner distal end in the retracted position;
Fig. 5 is a section similar to Fig. 4 with the stimulating tip in the operative forward position;
Figs. 6 and 7 are perspective views, in a much more enlarged scale than Figs. 4 and 5, with a cut-away portion of the wall of the threaded pipe to show the inside.

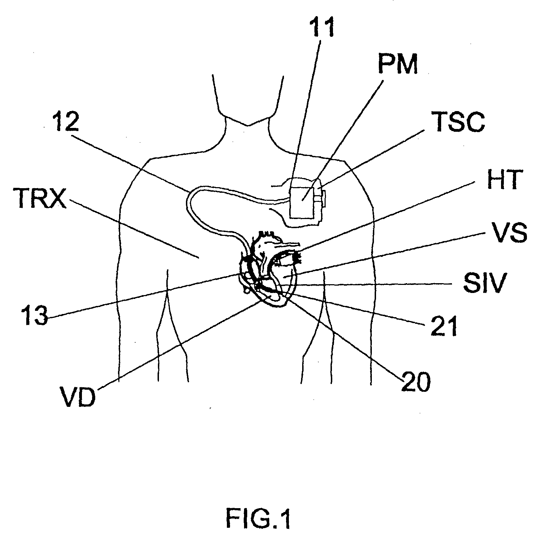

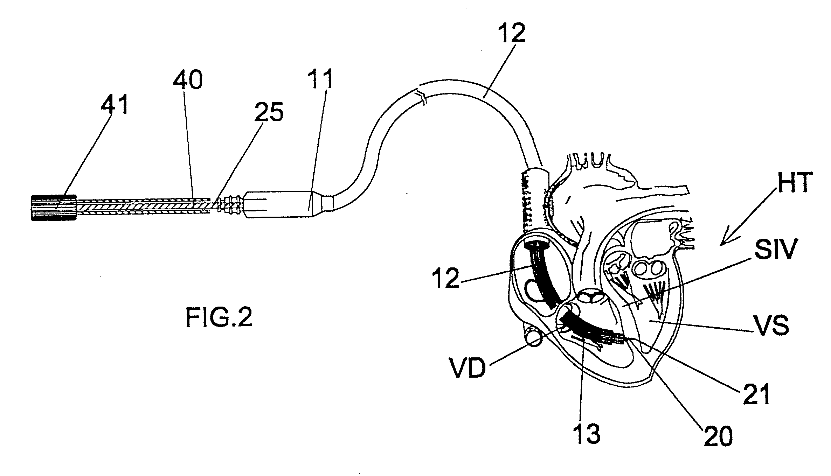

With reference firstly to Figs 1 and 2, it should be noted that the cardiac electrostimulation device provided with a catheter electrode according to the invention positioned in the chest of the patient includes a pacemaker PM put in pouch TSC and a catheter electrode 12 that leads the electrical pulses generated by pacemaker PM to the heart, at the end portion 13 of which the stimulating tip 20 with penetration point 21 is brought.

As known to those skilled in the art, the catheter electrodes for the assisted ventricular cardiac stimulation are currently formed essentially by an outside end portion 11 put into pacemaker PM, a thin elongated composite body 12, and a stimulating end portion 13 put into the right ventricle VD of heart HT, whose stimulating screw with helical conical shape enters the interventricular septum SIV so as to be anchored thereto, which tip has an axial length equal to about 1.5 mm.

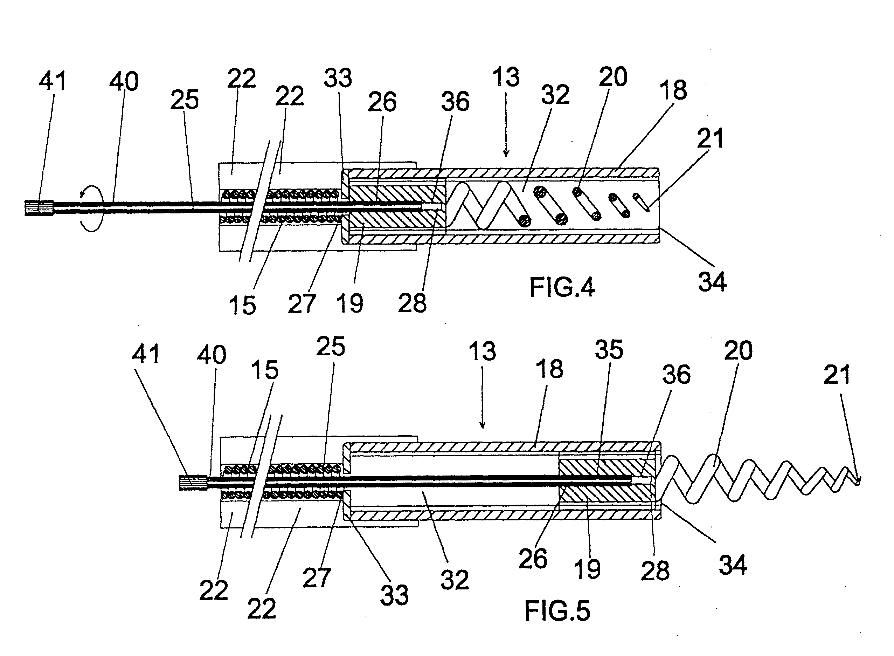

The elongated body 12 of the catheter electrode, as better seen thereafter, extends along several veins to reach heart HT from pouch TSC containing pacemaker PM. However, the catheter electrode consists of a very flexible helically wound "spring" 15 (Figs. 4 and 5) having coils contacting each other, and an also very flexible insulating sheath 22 made preferably by an elastomer which covers the whole spring. As a result, the electrode cannot be pushed inside the veins as mentioned above. Therefore, a quite flexible, however strong wire, so-called spindle indicated at 25, is used to position the catheter electrode 12 and is put into the bore of spring 15 of the catheter electrode to give the latter the right rigidity necessary to guide the catheter electrode to heart HT.

Such a rigidity, that the combination of the covering sheath and the electrode spring cannot provide, is actually necessary because of the path followed by the catheter electrode from the subcutaneous pouch TSC to heart HT and consisting in detail of left cephalic vein, left subclavian vein, superior vena cava, and at last atrium to enter the right ventricle VD through the tricuspid valve. A sufficient rigidity is provided by spindle 25 that, however, has a further function in the present invention, as better seen thereafter.

As already mentioned, the catheter electrode according to the invention has a stimulating end portion 13 electrically connected to the conductive helical spring 15, which end portion has a composite construction essentially consisting of a thin, inside threaded pipe 18 (like a nut screw), a cylindrical, outside threaded body 19 received in the internal bore 32 of cylindrical body 18 in screwing engagement, and a helically conical stimulating screw 20 having a penetration tip 21.

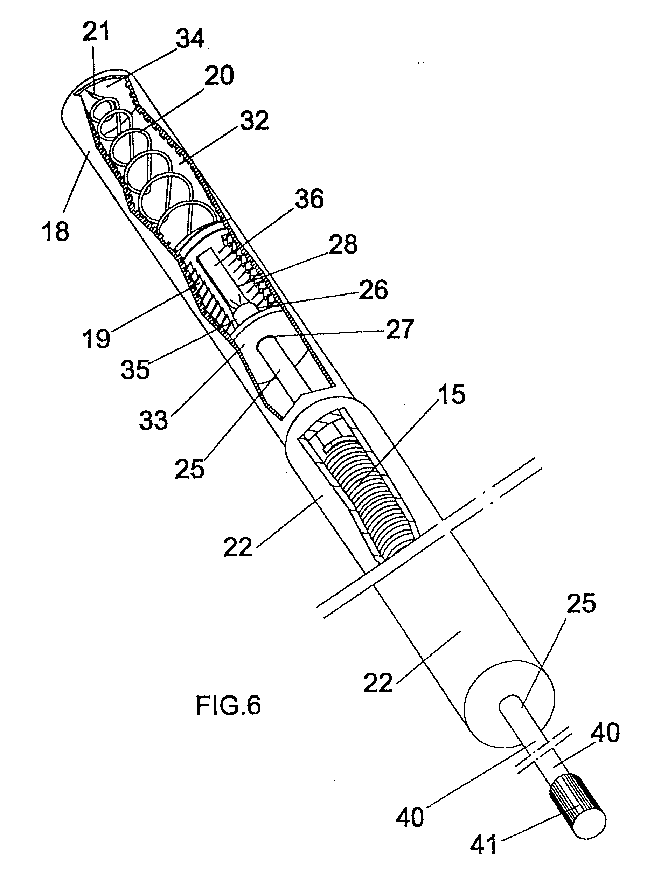

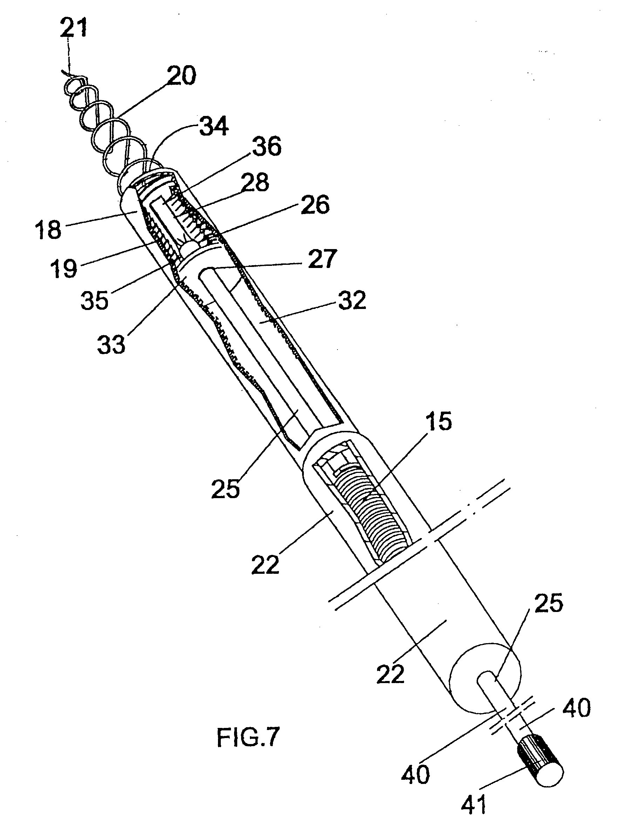

The threaded body 19 has an axial cavity 26 having a cylindrical mouth and an inside portion 28 with a squeezed rectangular section like a screw head. As can be seen in Figs. 4 to 7, spindle 25 has a short length with a generally circular section at the inside end 35 and a squeezed tip 36 like the blade tip of a screwdriver. Moreover, it carries a suitably knurled control knob 41 at the outside end 40.

The shape of the axial cavity 26, 27, 28 of the threaded body 19 and the shape of the distal end 35, 36 of the spindle are such that the end 36 of the spindle received in the squeezed cavity 28 can serve as "screwdriver" transmitting to threaded body 19 any rotation imparted from the outside to spindle 25 by operating knurled knob 41.

As clearly shown in Figs. 4 and 5, the axial size of cavity 32 inside pipe 18 is such that the cavity can receive both threaded body 19 and electrostimulating screw 20 with penetration tip 21 integral therewith when the threaded body 19 is in the most inside portion of such cavity contacting the circular wall 33 of pipe 18.

As in the catheter electrode according to the invention the stimulating screw to be anchored to interventricular septum SIV has an axial length of 8-10 mm, as already mentioned, and the threaded body is 4 mm long in the axial direction to receive both the stimulating screw and the threaded body, cavity 32 of pipe 18 should have a length of 15-16 mm.

Because of such a construction it is self-evident that the clockwise rotation of spindle 25 is transmitted to the threaded body 19 which is in non-reversible screwing engagement with the inside thread of pipe 18 so that it moves forward along the pipe and transmits both a straightaway motion towards the open end 34 of the pipe and a clockwise rotation to tip 20.

The operation of the stimulation device positioned and constructed as described above for the use of the single catheter electrode for biventricular stimulation is as follows:

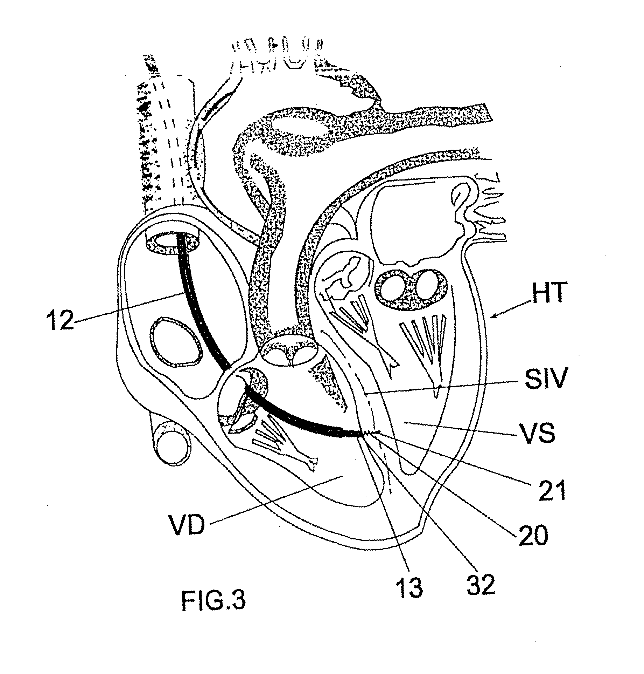

Once the stimulating end 13 is positioned in the right ventricle DV, as described above, with the annular portion 33 contacting the interventricular septum SIV (Figs. 2 and 3), spindle 25 is rotated in clockwise direction by knurled knob 41 so that the threaded body 19 can move forward by rotation in the cavity 32 under control of its feed in millimetres by the number of complete revolutions made by the spindle, as already mentioned. As it is self-evident, also the stimulating screw 20 will be fed by the same length by a simultaneous clockwise rotation. Therefore, screw 20 will enter deeply the interventricular septum SIV in its feed motion (Fig. 3) as such feed motion can be exactly controlled because it takes place millimetre by millimetre according to the number of complete revolutions of spindle 25. When tip 21 has overcome the middle plane of the interventricular septum SIV, as already described, one can control by the instrumentation when the stimulating screw 20 has entered the interventricular septum SIV by such a length as to electrically stimulate simultaneously right ventricle DV and left ventricle, i.e. when the biventricular stimulation is carried out. At this time the rotation of spindle 25 is interrupted and the spindle is extracted from spring 15.

It is self-evident from the foregoing that the catheter electrode of the present invention solves thoroughly and easily without complications the problem of providing a biventricular electrostimulation by using only one electrode.

## Claims

1. A single pacemaker catheter electrode for the biventricular cardiac electrostimulation which is part of an assisted cardiac stimulation pacemaker (PM) conceived and constructed so that its insertion into the right ventricle (VD) permits the biventricular electrostimulation, i.e. also the stimulation of the left ventricle (VS), without the need of using a second catheter electrode directed to stimulate the left ventricle, including: a positioning end portion (11) of the pacemaker (PM), an elongated body (12) consisting of a helical flexible spring (15) with coils very close to one another made of an electrically conductive material, an also very flexible electrically insulating sheath (22) covering the spring completely, a stiffening wire or spindle (25) located inside said spring (15) upon positioning said catheter electrode, and a stimulating end portion (13) to be put into said right ventricle (VD) in contact with the interventricular septum (SIV) of the heart, including a stimulating screw (20) having a penetration tip (21), **characterized in that** said electrostimulating end portion (13) has a composite construction consisting of a metal pipe (18) with an inside thread electrically connected to said conductive spring (15), a cylindrical body (19) having an outside thread conjugated with said inside thread and in screwing engagement therewith, and a helically conical stimulation screw (20) integral with said threaded body (19) which has an axial countersunk cavity (26, 27, 28) having differentiated sections, that the distal end (35, 36) of said spindle (25) has sections which are complementary to those of said cavity (26, 27, 28) so as to engage with each other against any relative rotation motion, and that said spindle has a proximal end (40) provided with a knurled knob (41) which facilitates the rotation of said spindle (25) about its own axis inside the cavity of said spring (15), the arrangement of the parts being such that the rotation of said spindle (25) causes said threaded body (19) to rotate and to move forward in the inside cavity (32) of said pipe (18), thus bringing said stimulating screw (29, 21) first into contact and then in screwing engagement with said interventricular septum (SIV) entering deeply therein.

2. The single pacemaker catheter electrode for the biventricular cardiac electrostimulation according to claim 1, wherein differentiated sections (27, 28) of said cavity (26) of said worm screw (19) and said differentiated sections (35, 36) of the end portion of said spindle (25) have a circular shape (27, 35) at the mouthpiece and a squeezed shape (28, 36) like a screwdriver tip near the countersink so that the insertion of said end into said cavity causes the integral engagement with each other.

3. The single pacemaker catheter electrode for the biventricular cardiac electrostimulation according to claim 2, wherein said threaded body (19) and said stimulating screw (20) are received in axial alignment in the inside cavity (32) of said pipe with inside thread (18) when they are in their completely retracted, non-operative position.

4. The single pacemaker catheter electrode for the biventricular cardiac electrostimulation according to claim 3, wherein said inside thread of said threaded pipe (18) and said outside thread of said threaded cylindrical body (19) have such a pitch that every millimetre feed of said cylindrical body corresponds to a whole number of revolutions of said spindle (25).

5. A biventricular cardiac electrostimulation with assisted stimulation obtained by inserting into the right ventricle (VS) of heart (HT) a single catheter electrode which is so shaped as to electrically stimulate simultaneously right ventricle (VD) and left ventricle (VS) by any single stimulation pulse generated by pacemaker (PM) associated thereto, such simultaneous electrostimulation being obtained by causing the helical stimulating screw (20, 21) carried by the end portion (13) of the catheter electrode to deeply penetrate into the interventricular septum (SIV), and in any case beyond the middle plane thereof, which screw (20, 21) is integral with a cylindrical body (19) received in said end portion (13) and driven from the outside by spindle (25) which is used to position the catheter electrode in the right ventricle (VD) of heart (HT) of the patient so as to perform a combined traverse and rotation motion bringing said stimulating screw (20, 21) outside said end portion (13) and to a deep screwing engagement with said interventricular septum (SIV).

6. The biventricular cardiac electrostimulation obtained by catheter electrode according to claim 5, wherein said penetration of said stimulating screw (20, 21) into said interventricular septum (SIV) is controlled from the outside by an electrocardiograph which displays the biventricular stimulation of heart (HT) of the patient at each single pulse of stimulation generated by said pacemaker (PM).

7. The biventricular cardiac electrostimulation obtained by catheter electrode according to claim 6, wherein the combined traverse-rotation motion of said cylindrical body (19) by said spindle (25) is performed in a controlled way millimetre by millimetre.
